Europäisches Patentamt

European Patent Office

Office européen des brevets

(19)

(11) EP 0 790 225 A1

(12) **EUROPEAN PATENT APPLICATION**

(43) Date of publication:
20.08.1997 Bulletin 1997/34

(21) Application number: 97660011.4

(22) Date of filing: 11.02.1997

(51) Int Cl.$^6$: **C07C 5/333**, B01J 23/22
// (B01J23/22, 101:32, 101:50, 103:18)

(84) Designated Contracting States:
**AT BE DE ES FR GB IT NL PT SE**

(30) Priority: 16.02.1996 FI 960698

(71) Applicant: NESTE OY
FIN-02150 Espoo (FI)

(72) Inventor: **Niemi, Vesa**
**06400 Porvoo (FI)**

(74) Representative: **Hakkila, Maini Annika et al**
**Forssén & Salomaa Oy,**
**Yrjönkatu 30**
**00100 Helsinki (FI)**

(54) **Process for the Dehydrogenation of alkanes and a catalyst for use therein**

(57) The object of the invention is a process and a catalyst for the dehydrogenation of alkanes, wherein the alkanes are contacted at an elevated temperature with a vanadium-containing catalyst in order to prepare alkenes. According to the invention, before the dehydrogenation reaction the catalyst is subjected to a carbon monoxide reduction, which improves the activity and selectivity of the catalyst.

The catalyst may contain modification metals, for example calcium or zirconium.

## Description

The invention relates to a process for the dehydrogenation of alkanes and to a catalyst for use therein. In particular in the process alkanes are contacted at an elevated temperature with a vanadium-containing catalyst reduced with carbon monoxide.

Current commercial dehydrogenation catalysts are based on the use of either chromium oxide or a platinum metal. However, chromium has numerous different degrees of oxidation, of which Cr(VI) is known to be carcinogenic and allergenic. Platinum, on the other hand, is a very expensive metal. For the above reasons, the use of catalysts causes difficulties in processes in which it is not possible to prevent completely the loss of catalyst (for example, the fluidized-bed process).

The present invention relates in particular to a method in which the currently used chromium dehydrogenation catalyst can be replaced with an equally active and equally selective but more environment-friendly catalyst.

The use of vanadium catalysts in dehydrogenation is known. For example, US patent 4,607,129 comprises the dehydrogenation and/or dehydrocyclization of alkanes and/or cycloalkanes by using a $V_2O_5/SiO_2$ catalyst. The catalyst is regenerated by contacting it with a gas which contains free oxygen, usually air, in conditions which promote the regeneration of dehydrogenation and/or dehydrocyclization. The examples of the patent deal with the dehydrogenation/ dehydrocyclization of n-octane and hexane and the dehydrogenation of cyclohexane, propane and isobutane.

US patent 4,644,089 describes a process in which alkanes/cycloalkanes are dehydro-genated/dehydrocyclized by using a catalyst of vanadium oxide and aluminium phosphate at the same process conditions as in the preceding patent. The catalyst is regenerated therein by contacting it with free oxygen at suitable regeneration conditions. In the process, alkanes and cycloalkanes (in which the number of carbon atoms is even up to 20) are converted, in the presence of the above-mentioned catalyst, into products which contain hydrogen gas and dehydrogenated and/or dehydrocyclized hydrocarbons. The catalyst contains, in addition to vanadium oxide, also small amounts of other metal oxides or sulphides, such as $MoO_3$, $NiO$, $MoS_3$, $NiS$, and the like.

US patent 5,220,092 describes a process for the preparation of alkenes, in which the catalyst used is vanadium oxide (4-9 % being $V_2O_5$) on a modified $\gamma$-$Al_2O_3$ support. In this process, a shorter contact period and a higher temperature are used than in the former one. Thus a significantly larger quantity of alkenes is obtained. The improvement in the dehydrogenation of non-branched alkanes is considerable, since non-branched alkanes are more difficult to dehydrogenate selectively than are branched alkanes. The examples of the patent deal with the dehydrogenation of propane and isobutane both by using unmodified $V/Al_2O_3$ catalysts and by using modified $V/X/Al_2O_3$ catalysts (X = Li, K. Cs, P).

The catalyst disclosed in EP patent application EP-0556589 is a modification of the foregoing patent. The objective has been to reduce the formation of coke by adding to the catalyst also small amounts of noble metals (Pt, Pd, Rh) or possibly Ge, Sn or Pb. Examples used are the dehydrogenation of isobutane at 650 °C and the dehydrogenation of propane at 700 °C with short retention periods. Phase changes of the support material occurring in catalyst regenerations are not mentioned in the patents.

The use of vanadium catalysts in oxidizing dehydrogenation is also known. In the oxidizing dehydrogenation the alkanes are dehydrogenated in the presence of an oxidizing component, for example of free oxygen. The hydrogen produced in the dehydrogenation is converted in the presence of oxygen into water vapour.

The present invention is based on oxygen-free dehydrogenation, wherein the catalyst, which is gradually deactivated owing to the coke formed in the dehydrogenation reaction, is regenerated by using an oxidizing gas, for example by air.

In general, the conversions and selectivities achieved by using vanadium catalysts are not as high as those achieved with chromium catalysts. On the other hand, chromium catalysts contain carcinogens and are therefore not especially attractive owing to the environmental risks. Therefore it would be desirable in the dehydrogenation of alkanes to replace these catalysts with more environment-friendly, vanadium-containing catalysts and, nevertheless, to achieve activities and selectivities similar to those achieved by using chromium catalysts.

A catalyst more environment-friendly than chromium catalysts could be used, for example, in a fluidized-bed reactor, in which the problems of heat transfer are easier to solve. In a fluidized-bed reactor it is possible to use a temperature higher than at present and to avoid thermal reactions by shortening the reaction period.

Thus the invention relates to a process in which vanadium catalysts are used and, nevertheless, conversions and selectivities are accomplished which are at the same level or at nearly the same level as those accomplished with commercial chromium catalysts. The process according to the invention for the dehydrogenation of alkanes, wherein alkanes are contacted with a vanadium-containing catalyst at an elevated temperature in order to prepare alkenes, is characterized in that a carbon monoxide reduction is performed on the catalyst before the dehydrogenation reaction.

Carbon monoxide reduction of catalysts is previously known, but it has not been known that it would have any significant effect on the dehydrogenation reactions of alkanes. Carbon monoxide pretreatment at ammoxidation reaction conditions, improving the activity of a vanadium oxide catalyst, is known from US patent 3,901,933. In this patent, the catalyst is used in the ammoxidation of aromatic hydrocarbons.

The vanadium catalysts applied in the present invention may be prepared by any known preparation method by which the desired vanadium concentrations are introduced onto a support. Dry or wet impregnation and adsorption or precipitation methods can be mentioned as examples. Of dry impregnation methods in particular, co- and successive impregnations can be mentioned, which have led to good results in terms of both dehydrogenation and of phase changes of the support. After the impregnation, adsorption or precipitation, the support is dried, for example at 120 °C, and is calcined. The calcination is carried out under an oxygen-containing atmosphere, in particular air, within a temperature range of 300-800 °C.

The recommended concentration of vanadium is 1-15 % by weight, in particular 2-5 % by weight, calculated as vanadium metal of the weight of the catalyst. The starting materials for the vanadium may be any vanadium compounds, for example ammonium vanadate. The solubility of ammonium vanadate can be improved by adding oxalic acid to the impregnation solution.

In addition to vanadium, the catalyst may contain one or several modification components. Recommended modification components include alkaline-earth metals, in particular calcium and strontium. The starting materials used for these components may likewise be water-soluble compounds of the respective metals, for example nitrates of alkaline-earth metals.

The modification agents used may also be elements of Group IV A, in particular zirconium; for example, zirconium nitrate can be used as its starting material. The ratio of the molar amount of vanadium impregnated onto a support to the molar amount of the alkaline-earth metal should be below 4. Furthermore, the ratio of the molar amount of vanadium to the metal of Group IV A should be below 2.

The support used may be any available support, of which in particular $\gamma$, $\theta$ or $\delta$ aluminium oxide, having a surface area of 50-400 m$^2$/g, is well suited for this purpose. Before the impregnation with vanadium the support may be stabilized thermally either without a modification component or with it.

The catalyst reduction according to the invention can be carried out by contacting the catalyst with a carbon monoxide atmosphere. If so desired, the carbon monoxide may be diluted with an inert gas, such as nitrogen. Alternatively, the reducing agent used may be, for example, a synthesis gas. The treatment temperature may be 400-800 °C, and the treatment period, for example, 0.01 minute - 2 hours. After the treatment the carbon monoxide may be rinsed off by using, for example, nitrogen gas.

The feed of the dehydrogenation process consists of one or several hydrocarbons which can be dehydrogenated. Typically the feed contains linear and/or branched $C_2$-$C_{10}$ alkanes, in particular, $C_2$-$C_5$ alkanes.

The dehydrogenation reaction can be carried out by using any reactor type, in which the alkane to be dehydrogenated is contacted at an elevated temperature with a catalyst. The reaction can be carried out in, for example, a fixed-bed reactor or a fluidized-bed reactor. A suitable reaction temperature is 500-700 °C. The pressure in the reactor may range from 0.1 to 5 bar. In addition to the alkane/alkanes fed in, also other gases, for example, nitrogen, water vapor, carbon monoxide or a synthesis gas, may be fed into the reactor.

In the performing of the examples below, a simplified trial hydrogenation apparatus was used, in which the reactor tubes were made of quartz glass, their inner diameter being 9 mm and length 60 cm. After charging of the catalyst, the reactor was filled with an inert silicon carbide to minimize the gas volume. The feed gas used was n-butane (> 99. 5 % by weight), which was diluted (1 mol/1 mol) with nitrogen (5.0). In the catalyst regenerations, synthetic air (4.8) (20 ml/min) diluted with nitrogen (110 ml/min) was used. The product mixture was analyzed by using an HP Series II gas chromatograph equipped with HP ChemStation software, $Al_2O_3$/KCB - PLOT column, and an FID detector.

The results were calculated from the molar percentages (without hydrogen) calculated from the product analysis, without taking into account the coke content remaining in the catalyst. The conversion X of n-butane is in this case obtained from the formula

$$(1) \qquad X(\%) = \{(\text{products - n-butane}) / \text{products}\} * 100.$$

The products fraction is based on the carbon number, corrected with the purity of the feed.

The selectivities S of the individual products are calculated using Formula (2)

$$(2) \qquad X(\%) = \{\text{product} / (\text{products - n-butane})\} * 100.$$

In the calculation of selectivity, the product is corrected, when necessary (ethylene, propylene), by using a carbon number coefficient of C/4, where C is the carbon number of the product. The yields of the product or of the product groups are further obtained from Formula (3):

(3)     $Y(\%) = X * S / 100.$

## Example 1

A catalyst was prepared by crushing (0.5-1.0 mm) the γ-aluminium oxide support (surface area approx. 200 m²/ g, pore volume 1.2 ml/g) and by a precalcination for 16 h at 750 °C. An aqueous solution prepared from ammonium vanadate ($NH_4VO_3$), oxalic acid ($(COOH)_2 \cdot 2H_2O$) (molar ratio of $NH_4VO_3$ to $(COOH)_2 \cdot 2H_2O$ being 0.5) and calcium nitrate was co-impregnated by dry-impregnation onto the support. The catalyst was aged for 1 h at room temperature, was dried for 4 h at 120 °C, and was calcined for 2 h at 700°C (temperature increase being 2 °C/min). The calculated vanadium content of the catalyst was 5 % by weight, the V/Ca molar ratio was 3, and the support was γ-vanadium oxide.

The dehydrogenation was carried out by charging with 500 mg of the catalyst obtained above a quartz glass reactor (inner diameter 9 mm, length 60 cm) filled with silicon carbide. The catalyst bed was heated to 650 °C under a nitrogen stream (quality 5,0, 1 l/h). After the stabilizing of the temperature, n-butane (<99.5 % by weight) (n-$C_4$/$N_2$ molar ratio being 1) was added to the feed. A WHSV value of 12h⁻¹ was used as the feed rate for n-butane, whereupon approx. 0.4 s was obtained as the contact period. The product sample for gas chromatography was taken 20 seconds after initiation of the n-butane feeding. On the basis of this, the conversion of n-butane and product selectivities were calculated. After the taking of the sample, the feeding in of n-butane was discontinued, the catalyst was stripped with nitrogen and it was regenerated with diluted air (air 20 ml/min + nitrogen 110 ml/min). After regeneration, repeat experiments were carried out. Preliminary reduction was carried out by using carbon monoxide (> 99 %) or by hydrogen (quality 5.0) (5 min approx. 20 ml/min), and thereafter, before the reaction, nitrogen stripping of the catalyst was carried out.

In Experiment 1, the dehydrogenation reaction of n-butane was carried out without the reduction of the catalyst. In Experiment 2, a hydrogen reduction was first carried out on the catalyst before the dehydrogenation reaction. In Experiment 3, reduction of the catalyst with carbon monoxide was carried out in accordance with the invention before the dehydrogenation. For comparison, a dehydrogenation reaction was carried out by using a chromium catalyst (10 % Cr), Experiment 4. The results are shown in accompanying Table 1. All of the experiments were carried out at 650 °C with a retention period of approx. 0.4 s.

Table 1

| Experiment | Pretreatment | Conversion (%) | Selectivities (%) | | | Olefin yield (%) |
|---|---|---|---|---|---|---|
| | | | n-butenes | i-butenes | olefins | |
| 1 | - | 48.7 | 51.9 | 2.0 | 86.1 | 41.9 |
| 2 | H$_2$ reduction | 32.7 | 64.4 | 2.1 | 91.1 | 36.5 |
| 3 | CO reduction | 65.4 | 65.5 | 2.9 | 90.9 | 59.5 |
| 4 | - | 68.0 | 63.0 | 0.5 | 90.0 | 62.0 |
| n-butenes contain: | | 1-, trans-2, cis-2-butene | | | | |
| olefins contain: | | ethylene, propylene, 1-, trans-2- and cis-2-butene, butadiene and i-butene | | | | |

The carbon monoxide reduction activates the V 5 %/Ca 3:1/Al$_2$O$_3$ catalyst considerably, and values corresponding to those obtained with a chromium catalyst are achieved in conversion and selectivities. It is seen from the results that H$_2$ reduction increases selectivity (primarily that of n-butenes), but the activity of the catalyst is decreased (from 49 % to 32 %). CO reduction, on the other hand, increases activity (from 49 % to 65 %) and selectivity, in which case an olefin yield of almost 60 % is achieved. A higher i-butene selectivity (2.9 vs. 0.5 %) and a lower butadiene selectivity (12.7 vs. 16.9 %) can be deemed to be advantages of a CO-reduced V 5 %/Ca 3:1/Al$_2$O$_3$ catalyst over a Cr catalyst.

## Example 2

A successive impregnation method was applied in the adding of the Group IV A modification component. An aqueous solution of zirconium oxynitrate (starting material $ZrO(NO_3)_2xH_2O$) was dry-impregnated onto the above-mentioned pre-calcinated support. After 1 h of ageing the modified support was dried at 120 °C for 4 h, whereafter the above-mentioned mixture of ammonium vanadate and oxalic acid was dry-impregnated. The catalyst was aged at room temperature for 1 h, whereafter the catalyst was dried and calcined as above.

A vanadium catalyst prepared by the above method, having a vanadium concentration of 2.6 % by weight and a V/Zr molar ratio of 1, γ-vanadium oxide as the support, was applied to the dehydrogenation reaction of n-butane in the above manner. The dehydrogenation was carried out at 650 °C, the retention period being approx. 0.4 s. The results are shown in Table 2.

Table 2

| Pretreatment | Conversion (%) | Selectivities (%) | | | Olefin yield (%) |
|---|---|---|---|---|---|
| | | n-butenes | i-butene | olefins | |
| - | 42.9 | 47.9 | 5.3 | 84.6 | 36.3 |
| H$_2$ reduction | 46.6 | 54.4 | 11.8 | 89.2 | 41.6 |
| CO reduction | 62.7 | 57.3 | 9.9 | 88.7 | 55.7 |
| n-butenes contain: 1-, trans-2- and cis-2-butene | | | | | |
| olefins contain: ethylene, propylene, 1-, trans-2- and cis-2-butene, butadiene and i-butene | | | | | |

Also in this case, the advantageous effect of carbon monoxide reduction on the conversion and the olefin yield is to be noted.

Claims

1. A process for the dehydrogenation of alkanes, wherein alkanes are contacted at an elevated temperature with a vanadium-containing catalyst in order to prepare alkenes, **characterized** in that, before the hydrogenation reaction, a carbon monoxide reduction is carried out on the catalyst.

2. A process according to Claim 1, **characterized** in that the reduction is carried out by contacting the catalyst with an atmosphere which contains carbon monoxide, at 450-800 °C for 0.01 min - 2 hours.

3. A process according to Claim 1 or 2, **characterized** in that the vanadium concentration of the catalyst is 1-15 % by weight, calculated from the weight of the catalyst.

4. A process according to any of the above Claims, **characterized** in that any support known per se is used as a support material, particularly γ-, θ- or δ-aluminium oxide.

5. A process according to any of the above Claims, **characterized** in that the catalyst contains one or more modification metals selected from a group consisting of alkaline-earth metals and Group IV A metals.

6. A process according to Claim 4, **characterized** in that the modification agent is calcium and that the V/Ca ratio in the catalyst is below 4.

7. A process according to Claim 4, **characterized** in that the modification agent is zirconium and that the V/Zr ratio in the catalyst is below 2.

8. A process according to any of the above Claims, **characterized** in that the dehydrogenation reaction is carried out at a temperature of 500-700 °C.

9. A catalyst for the dehydrogenation of alkanes at an elevated temperature to prepare alkenes, which catalyst contains a vanadium compound, **characterized** in that, before the dehydrogenation reaction, the catalyst has been contacted with an atmosphere which contains carbon monoxide.

10. A catalyst according to Claim 8, **characterized** in that its vanadium concentration is 1-15 % by weight, calculated from the weight of the catalyst.

11. A catalyst according to Claim 8 or 9, characterized in that the support material used is any support known per se, in particular γ-, θ- or δ-aluminium oxide.

12. A catalyst according to any of the above Claims 8-10, **characterized** in that the catalyst contains one or several modification metals selected from a group consisting of alkaline-earth metals and Group IV A metals.

13. A catalyst according to Claim 11, **characterized** in that the modification agent is calcium and that the V/Ca ratio in the catalyst is below 4.

14. A catalyst according to Claim 11, **characterized** in that the modification agent is zirconium and that the V/Zr ratio in the catalyst is below 2.

European Patent
Office

# EUROPEAN SEARCH REPORT

Application Number

EP 97 66 0011

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DE 18 15 773 A (M. FREMERY ET AL) 24 July 1969 | 1-14 | C07C5/333 B01J23/22 //(B01J23/22, 101:32,101:50, 103:18) |
| Y | * claims * <br> * page 4, paragraph 2 * <br> * table 1 * | 1 | |
| D,Y | US 3 901 933 A (NORTON RICHARD V) 26 August 1975 <br> * claims * | 1 | |

---

-----

TECHNICAL FIELDS
SEARCHED     (Int.Cl.6)

C07C
B01J

The present search report has been drawn up for all claims

| Place of search | Date of completion of the search | Examiner |
|---|---|---|
| THE HAGUE | 29 April 1997 | Van Geyt, J |

EPO FORM 1503 03.82 (P04C01)